# EUROPEAN PATENT APPLICATION

(11) **EP 0 926 216 A1**
(43) Date of publication of application: **30.06.1999**
(21) Application number: 98309831.0
(22) Date of filing: 01.12.1998
(51) Int. Cl.: C09K 11/06, H05B 33/14, C07C 211/55

(54) **Organic electroluminescent device using a triarylamine derivative**

(30) Priority: 01.12.1997 JP 34579697; 28.09.1998 JP 27329398
(71) Applicant: CHISSO CORPORATION, Osaka-shi Osaka 530-6591 (JP)
(72) Inventor: Uchida, Manabu, Yokohama, Kanagawa, 236-0024 (JP); Koike, Toshihiro, Yokohama, Kanagawa, 236-0024 (JP); Izumizawa, Takenori, Yokohama, Kanagawa, 236-0024 (JP); Furukawa, Kenji, Yokosuka, Kanagawa, 239-0831 (JP)
(74) Representative: Ruffles, Graham Keith

(57) **Abstract**

A luminescent material, particularly an organic luminescent material, containing a triarylamine derivative of formula (1): 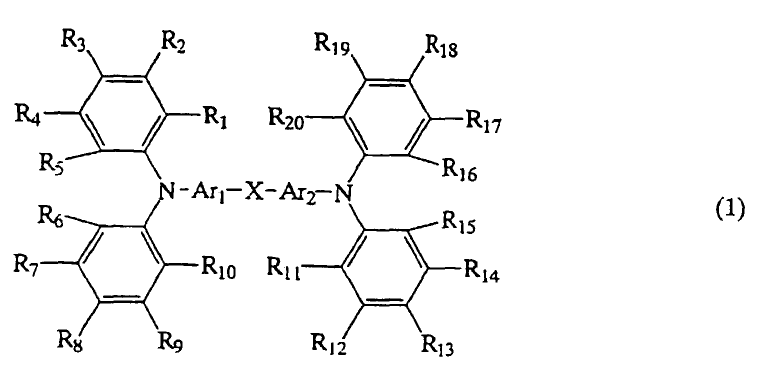 wherein X represents an arylene group other than a phenylene group, or a divalent heterocyclic group other than a thienylene group; Ar₁ and Ar₂ independently and individually represent a phenylene group or a naphthylene group; and R₁ through R₂₀ independently and individually represent a hydrogen atom, a halogen atom, a C1-C6 alkyl group, a C1-C6 alkoxy group, a substituted or unsubstituted amino group, a substituted or unsubstituted aryl group, or a substituted or unsubstituted heterocyclic group, the substituted or unsubstituted aryl group or the substituted or unsubstituted heterocyclic group may be condensed with the corresponding benzen ring at an arbitrary position of the benzen ring. Also disclosed is an organic electroluminescent device (organic EL device) making use of the above compound. The organic electroluminescent device has a long service life and exhibits high luminous efficiency.

## Description

### BACKGROUND OF THE INVENTION

### Field of the Invention

The present invention relates to an electroluminescent material using a triarylamine derivative, particularly an organic electroluminescent material, as well as to an organic electroluminescent device (hereinafter abbreviated as an organic EL device).

### Background Art

In recent years, organic EL devices have become of interest as candidates for flat displays of high brightness. In accordance with this trend much research and development therefor has been actively performed. An organic EL device has a structure in which a luminescence layer is sandwiched by two electrodes. Holes injected from an anode and electrons injected from a cathode are united within the luminescence layer to thereby emit light. High- and low-molecular-weight materials may both be used for fabrication of organic EL devices, and both have proven to provide organic EL devices of high brightness.

Organic EL devices are categorized into two types. One type utilizes a fluorescent-dye-added charge transportation material to form a luminescence layer (C. W. Tang, Journal of Applied Physics, 65, 3610, 1989), and the other type employs a fluorescent dye *per se* to serve as the luminescence layer (see, for example, Japanese Journal of the Applied Physics, 27, L269, 1988).

Organic EL devices using a fluorescent dye *per se* for the luminescence layer are further grouped into the following three types. A first type is drawn to three-layered devices in which a luminescence layer is sandwiched by a hole-transportation layer and an electron-transportation layer; a second type is drawn to two-layered devices in which a hole-transportation layer and a luminescence layer are superposed one on the other; and a third type is drawn to two-layered devices in which an electron transportation layer and a luminescence layer are superposed one on the other. Thus, organic EL devices are known to have improved luminous efficiency when they have a two- or three-layered structure.

In the above-mentioned organic EL devices, the electron transportation layer contains an electron-transfer compound and has a function of transferring electrons injected from a negative electrode into the luminescence layer. The hole-injection layer and the hole-transportation layer both contain a hole-transfer compound and have a function of transferring holes injected from an anode into the luminescence layer. When the hole-injection layer is interposed between the anode and the luminescence layer, organic EL devices having excellent luminous performance can be realized, because an increased number of holes can be injected into the luminescence layer at a lower electric field, and in addition, electrons injected from the cathode or electron-injection layer can be confined within the luminescence layer, to thereby enhance the luminous efficiency.

However, the aforementioned conventional organic EL devices do not necessarily exhibit sufficient performance in practical application. One major reason for this may be attributed to lack of durability of the material of the devices, particularly, hole-transportation material. It has been accepted that, if an irregular portion such as a grain boundary exists in the organic layer of an organic EL device, the electric field concentrates to such a portion to thereby lead to degradation and breakage of the device. Therefore, the organic layer is usually used in its amorphous state. Moreover, since an organic EL device is a current-injection-type device, if the material used has a low glass transition point, heat generated during use causes degradation of the organic EL device. From this point, materials having a high glass transition temperature (Tg) are desired.

Furthermore, hole-transportation materials used for conventional devices have insufficient hole transportability and therefore the luminous efficiency of the devices has not been satisfactory in practice.

A variety of materials centering on triphenylamine derivatives have been known as hole-transportation materials used for such organic EL devices, yet very few materials are suitable for practical use.

For example, there has been known N,N'-diphenyl-N',N'-di(3-methylphenyl)-4,4'-diaminobiphenyl (hereafter abbreviated as TPD) (Applied Physics Letter, Vol. 57, No. 6, page 531, 1990). This compound is thermally unstable, and has disadvantages in service life of the resultant device. Many other triphenylamine derivatives are disclosed in, for example, U.S. Patent Nos. 5047687, 4047948, and 4536457; Japanese Patent Publication (*kokoku*) No. 6-32307; and Japanese Patent Application Laid-Open (*kokai*) Nos. 5-234681, 5-239455, 8-87122, and 8-259940. However, none of them are satisfactory in terms of their characteristics.

Star-burst amine derivatives disclosed in Japanese Patent Application Laid-Open (*kokai*) No. 4-308688 or 6-1972, or "Advanced Material" Vol. 6, page 677 (1994) do not meet essential requirements for organic EL devices in practice, i.e., high luminous efficiency and long service life; and neither do respective compounds disclosed in Japanese Patent Application Laid-Open (*kokai*) Nos. 7-126226, 7-126615, 7-331238, 7-97355, 8-48656, and 8-100172, and "Journal of the Chemical Society Chemical Communication" page 2175 (1996).

The compounds having a thiophene ring disclosed in "Advanced Material" Vol. 9, page 720 (1997) have a drawback that they emit light having a long wavelength.

Japanese Patent Application Laid-Open (*kokai*) No. 62-201446 discloses a triarylamine derivative. However, this publication is silent about use of this compound in organic EL devices.

As described above, hole-transportation materials used in conventional organic EL devices still require improved performance, and thus there is need for an excellent material that can enhance the luminous efficiency and service life of organic EL devices.

### SUMMARY OF THE INVENTION

In view of the foregoing, the inventors of the present invention have conducted extensive studies in an attempt to solve the aforementioned problems involved in conventional organic EL devices, and have found that when a specific type of triarylamine derivatives is used, the purposes are successfully attained, leading to completion of the present invention.

Accordingly, an object of the present invention is to provide an organic EL device having high luminous efficiency and a prolonged service life.

Another object of the present invention is to provide a novel hole-transportation material used in the device.

A further object of the present invention is to provide a novel organic electroluminescent material used in the device.

In a first aspect of the present invention, there is provided a luminescent material comprising a triarylamine derivative of formula (1): 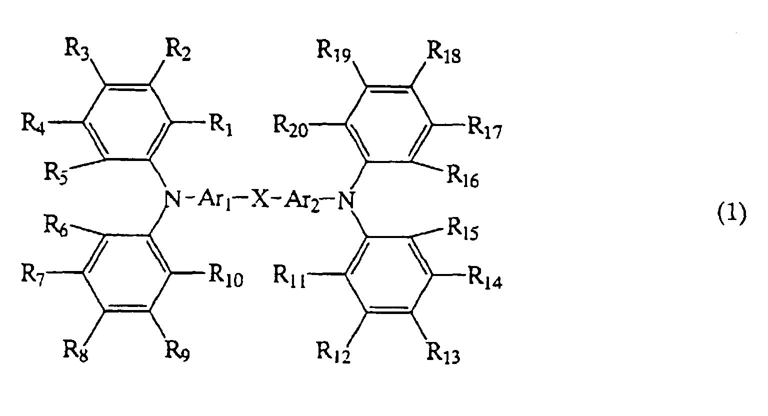 wherein X represents an arylene group other than a phenylene group, or a divalent heterocyclic group other than a thienylene group; Ar₁ and Ar₂ independently and individually represent a phenylene group or a naphthylene group; and R₁ through R₂₀ independently and individually represent a hydrogen atom, a halogen atom, a C1-C6 alkyl group, a C1-C6 alkoxy group, a substituted or unsubstituted amino group, a substituted or unsubstituted aryl group, or a substituted or unsubstituted heterocyclic group, the substituted or unsubstituted aryl group or the substituted or unsubstituted heterocyclic group may be condensed with the corresponding benzen ring at an arbitrary position of the benzen ring.

In a second aspect of the present invention, there is provided an organic electroluminescent material formed of the triarylamine derivative of formula (1).

In a third aspect of the present invention, there is provided an organic electroluminescent device comprising the triarylamine derivative of formula (1).

In a fourth aspect of the present invention, there is provided an organic electroluminescent device comprising a hole-transportation layer, wherein the hole-transportation layer contains the triarylamine derivative of formula (1).

In a fifth aspect of the present invention, there is provided an organic electroluminescent device comprising a luminescence layer, wherein the luminescence layer contains the triarylamine derivative of formula (1).

In a sixth aspect of the present invention, there is provided an organic electroluminescent device comprising a hole-injection layer, wherein the hole-injection layer contains the triarylamine derivative of formula (1).

In formula (1), when X is an arylene group, specific examples of the arylene include a naphthylene group, an anthrylene group, a biphenylene group, a toluylene group, a pyrenylene group, a perynylene group, an anisylene group, a terphenylene group, a phenanthrylene group, or a xylylene group. In this connection, if X in formula (1) is a phenylene group, the corresponding compounds have low luminous efficiency and thus are impractical.

When X in formula (1) is a divalent heterocyclic group, specific examples of the divalent heterocyclic group include a hydrofurylene group, a hydro pyrenylene group, a dioxanylene group, a furylene group, an oxazoylene group, an oxadiazolylene group, a thiazolylene group, a thiaziazolylene group, an acridinylene group, a quinolylene group, a quinoxaloylene group, a phenanthrolylene group, a benzothienylene group, a benzothiazolylene group, an indolylene group, a silacyclopentadienylene group, and a pyridylene group. In this connection, if X in formula (1) is a thienylene group, the corresponding compounds emit light having a longer wavelength and thus are impractical.

According to the present invention, a triarylamine derivative of formula (1) is a novel luminescent material having a high glass transition point; and is a novel organic electroluminescent material having excellent hole-injection performance, hole-transportation performance, and an electron-confinement effect. The use of the derivative can provide an organic EL device having high luminous efficiency and a long service life. Also, a highly effective display apparatus, such as a full color display, can be produced by the use of the organic EL device of the present invention.

### DESCRIPTION OF PREFERRED EMBODIMENTS

The present invention will next be described in detail.

Specific examples of the triarylamine derivatives of formula (1) include those represented by the following formulas (2) through (13):

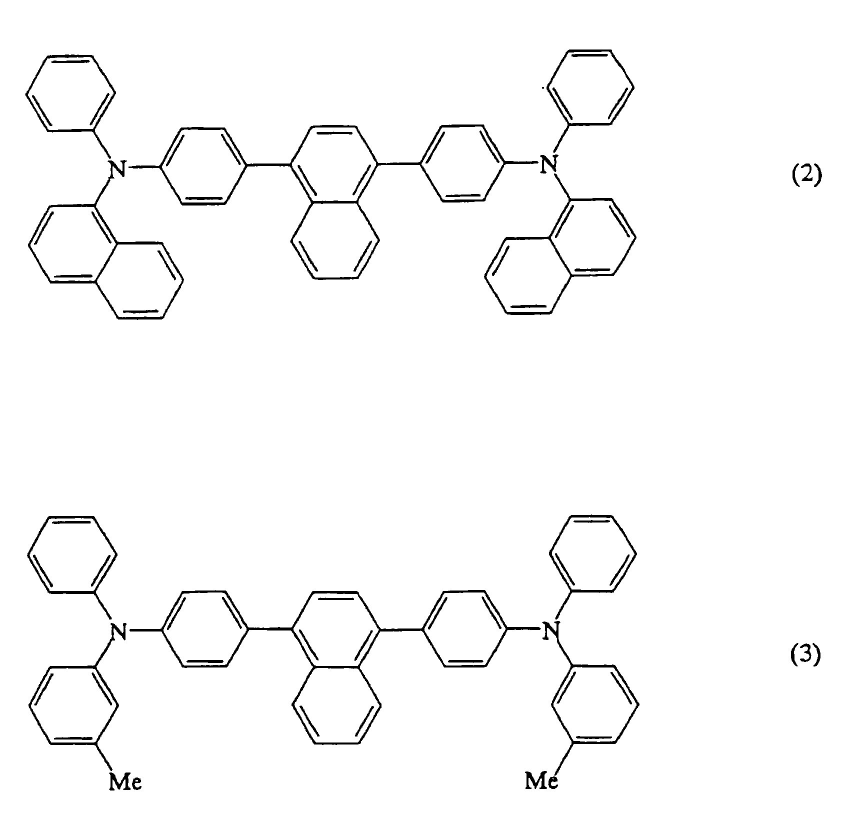

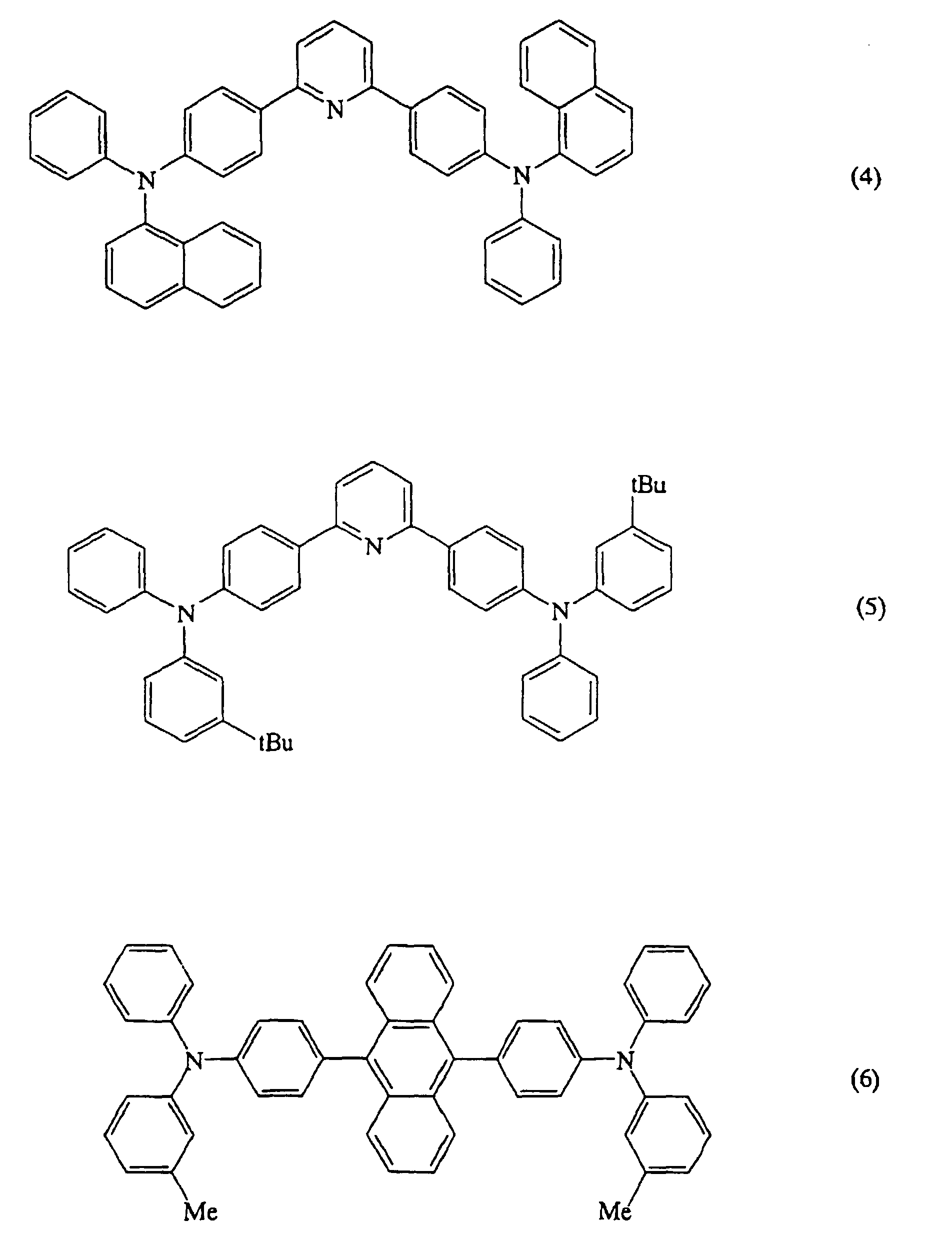

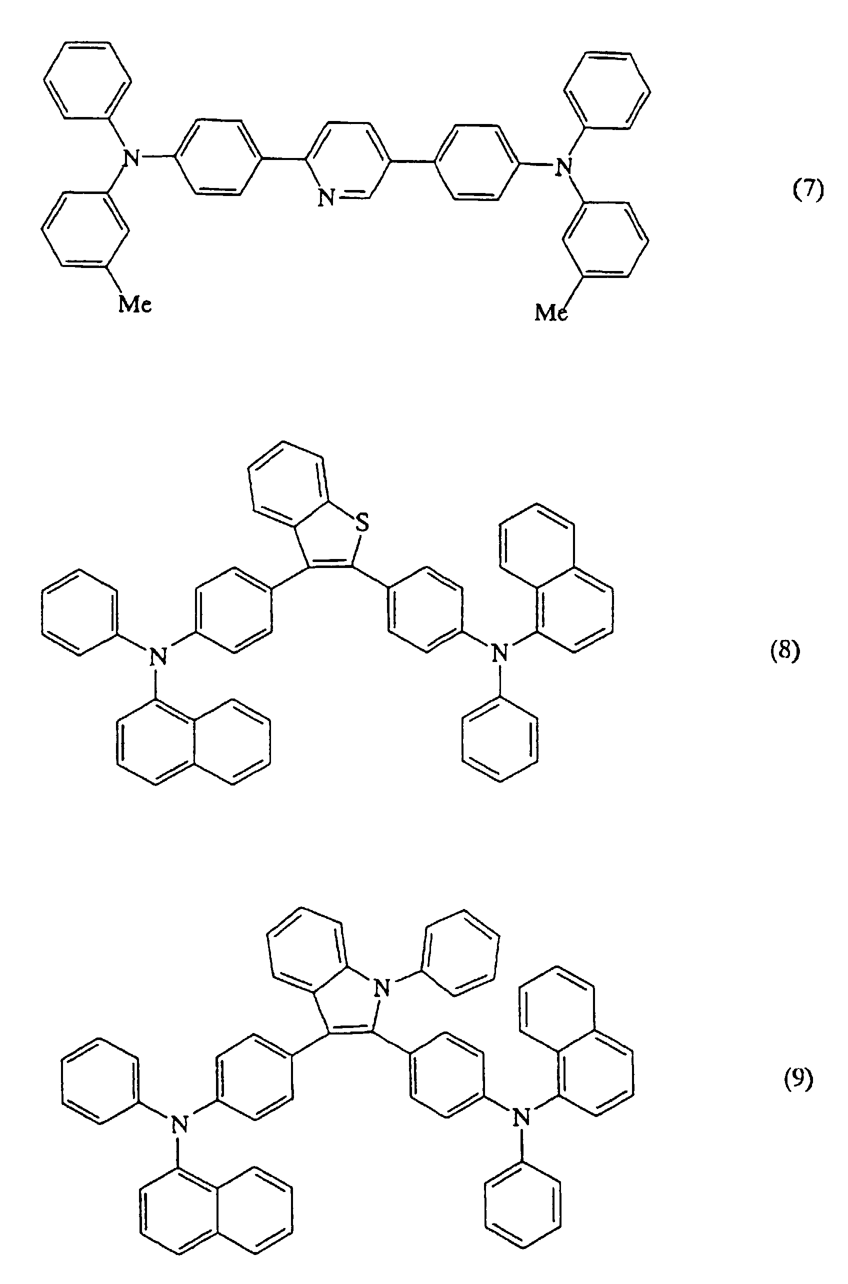

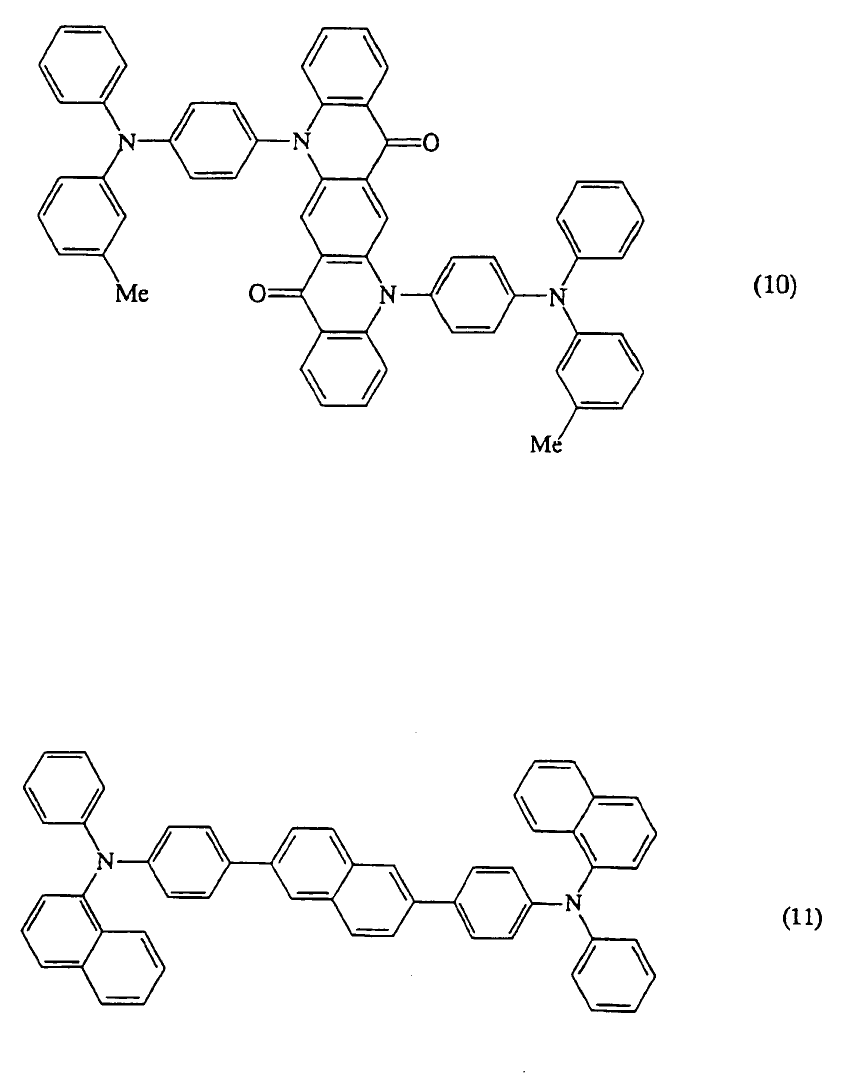

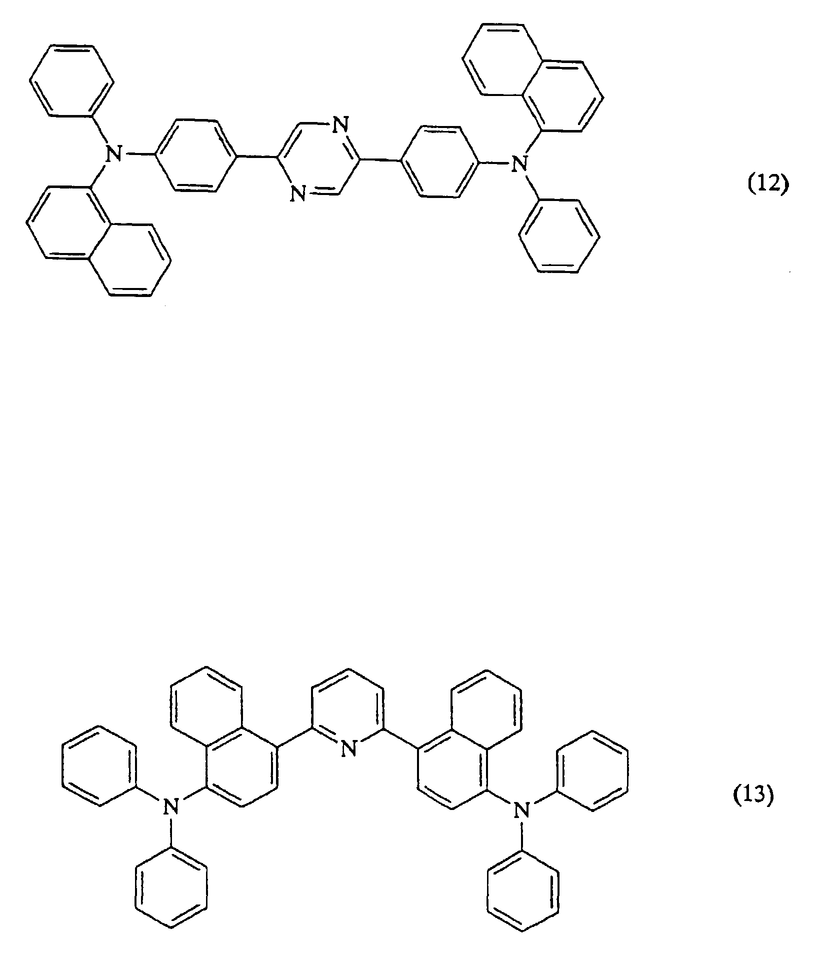

These triarylamine derivatives may be synthesized through a known synthesis method; for example, the methods described in Synthesis Examples of the present specification.

These triarylamine derivatives are suitable for luminescent materials because of their self-fluorescence emission, which is induced by introduction of an arylene group or a divalent heterocyclic group between two aniline moieties. In particular, these triarylamine derivatives glow blue, and provide organic EL devices having different emission colors by incorporation of another luminescent material such as a blue, green, or red luminescent material.

In general, a compound used in an organic layer that constitutes an organic EL device preferably forms no excited complex with a compound used in another layer. The triarylamine derivatives according to the present invention have an advantage that they do not easily form an excited complex with another compound. This is considered to be due to introduction of an arylene group or a divalent heterocyclic group between two aniline moieties.

The triarylamine derivatives represented by formula (1) function as both a hole-transportation material and a hole-injection material.

The organic EL device of the present invention may be realized in a variety of modes. Basically, the device has a structure wherein an organic layer containing the above-described triarylamine derivative is sandwiched by a pair of electrodes (anode and cathode), and, optionally, a hole-injection material, a hole-transportation material, a luminescent material, an electron-injection material, or an electron-transportation material may be incorporated into the above-described triarylamine derivative layer. When the triarylamine derivative layer serves as a luminescence layer, another luminescent material may be incorporated thereto, to thereby emit light having a different wavelength and enhance the luminous efficiency of the layer.

Alternatively, a hole-injection material, a hole-transportation material, a luminescent material, an electron-injection material, or an electron-transportation material may be formed into a layer and laminated on the triarylamine derivative layer.

Specific structural examples include lamination structures such as (1) node/triarylamine derivative layer/cathode; (2) anode/triarylamine derivative layer/luminescence layer/cathode; (3) anode/triarylamine derivative layer/luminescence layer/electron-injection layer/cathode; (4) anode/hole-injection layer/triarylamine derivative layer/luminescence layer/electron-injection layer/cathode; (5) anode/triarylamine derivative layer/hole-transportation layer/luminescence layer/electron-injection layer/cathode; and (6) anode/hole-injection layer/triarylamine derivative layer/electron-injection layer/cathode.

In the above cases, a hole-injection layer and an electron-injection layer are not always necessary; however, incorporation thereof enhances luminous efficiency of the EL device.

The organic EL device of the present invention, having whichever of the structures listed above, is preferably supported by a substrate. No particular limitation is imposed on the substrate, and glass, transparent plastic film, etc. may be used insofar as the material of the substrate has good mechanical strength, thermal stability, and transparency.

With regard to the anode materials of the organic EL device of the present invention, there may be used a metal, an alloy, an electrically conductive compound, or a mixture thereof having a work function of more than 4 eV. Examples include metals such as Au; and conductive transparent materials such as CuI, indium tin oxide (hereinafter abbreviated as ITO), SnO₂, or ZnO.

With regard to the cathode materials, there may be used a metal, an alloy, an electrically conductive compound, or a mixture thereof having a work function of less than 4 eV. Examples include calcium, magnesium, lithium, aluminum, magnesium alloys, lithium alloys, aluminum alloys, and other alloys such as aluminum/lithium, magnesium/silver, or magnesium/indium.

In order to output the luminescence of an organic EL device at a high efficiency, at least one electrode preferably has an optical transparency of 10% or more. The sheet resistance of the electrodes is preferably some hundreds of Ω/mm or less. The thickness of the electrodes, which depends on the electrode materials, is typically selected from 10 nm-1 µm, preferably 10-400 nm. Such electrodes may be produced by forming a thin film through vapor deposition or sputtering and by use of the above-described electrode materials.

With regard to a hole-injection material and hole-transportation material, in addition to the triarylamine derivatives that are used in the organic EL device of the present invention, there may be used an arbitrary material selected from materials which have conventionally been used as charge-transportation materials for holes among photoconductive materials and from known materials which are used in a hole-injection layer or a hole-transportation layer of an organic EL device.

Examples include carbazole derivatives (e.g., N-phenylcarbazole and polyvinylcarbazole); triarylamine derivatives (e.g., TPD, polymers having an aromatic tertiary amine in a main or side chain, 1,1-bis(4-di-p-tolylaminophenyl)cyclohexane, N,N'-diphenyl-N,N'-dinaphthyl-4,4'-diaminobiphenyl, 4,4',4'-tris{N-(3-methylphenyl)-N-phenylamino}triphenylamine, a compound described in "Journal of the Chemical Society Chemical Communication" page 2175 (1996), compounds described in Japanese Patent Application Laid-Open (*kokai*) Nos. 57-144558, 61-62038, 61-124949, 61-134354, 61-134355, 61-112164, 4-308688, 6-312979, 6-267658, 7-90256, 7-97355, 6-1972, 7-126226, 7-126615, 7-331238, 8-100172, and 8-48656); a star-burst amine derivative described in "Advanced Materials" Vol. 6, page 677 (1994)); stilbene derivatives (e.g., a compound described in Proceedings (II) of the 72th annual spring convention of The Chemical Society of Japan); phthalocyanine derivatives (e.g., metal-free phthalocyanine, copper phthalocyanine); and polysilane.

Each of a hole-injection layer and a hole-transportation layer of the organic EL device of the present invention may be formed of a single layer containing one or more species of the above-described compounds, or may comprise a plurality of layers laminated one on another, wherein the layers contain different species of compounds.

No particular limitation is imposed on the electron-injection material and the electron-transportation material that are used in the organic EL device of the present invention, and there may be used an arbitrary material selected from materials which have conventionally been used as charge-transportation materials among photoconductive materials and from known materials which are used in an electron-injection layer and an electron-transportation layer of organic EL devices.

Examples of such electron-transfer compounds include diphenylquinone derivatives (e.g., described in *Denshi-shashin Gakkai-shi,* 30, 3 (1991)); perylene derivatives (e.g., described in J. Apply. Phys., 27, 296 (1988)); oxadiazole derivatives (e.g., described in the above-described literature, Jpn. J. Appl. Phys., 27, L713 (1988), or Appl. Phys. Lett., 55, 1489 (1989)); thiophene derivatives (e.g., described in Japanese Patent Application (*kokai*) No. 4-212286); triazole derivatives (e.g., described in Jpn. J. Appl. Phys., 32, L917 (1993)); thiadiazole derivatives (e.g., described in the 43th Proceedings of The Society of Polymer Science, Japan, (III) Pla007)); metal complexes of an oxine derivative (the technical research report of *Denshi Jyoho Tsushin Gakkai*, 92(311), 43(1992)); polymers of a quinoxaline derivative (e.g., described in Jpn. J. Appl. Phys., 33, L250 (1994)); and phenanthroline derivatives (e.g., described in the 43th Proceedings of *Kobunshi Toronkai* 14J07).

With regard to another luminescent material used in the organic EL device of the present invention other than the triarylamine derivative, there may be used known luminescent materials, such as daylight fluorescent materials, fluorescent brighteners, laser dyes, organic scintillators, and reagents for fluorescent analysis, as described in "*Hikari Kinou Zairyo*" in *Kobunshi Kinou Zairyo* series, published by Kyoritsu Shuppan (1991), P236, edited by The Society of Polymer Science, Japan.

Specifically, preferable examples of the luminescent material include polycondensed ring compounds such as anthracene, phenanthrene, pyrene, chrysene, perylene, coronene, rubrene, and quinacridone; oligophenylene compounds such as quaterphenyl; scintillators for liquid scintillation such as 1,4-bis(2-methylstyryl)benzene, 1,4-bis(4-methylstyryl)benzene, 1,4-bis(4-methyl-5-phenyl-2-oxazolyl)benzene, 1,4-bis(5-phenyl-2-oxazolyl)benzene, 2,5-bis(5-tert-butyl-2-benzoxazolyl)thiophene, 1,4-diphenyl-1,3-butadiene, 1,6-diphenyl-1,3,5-hexatriene, or 1,1,4,4-tetraphenyl-1,3-butadiene; a metal complex of an oxine derivative described in Japanese Patent Application (*kokai*) No. 63-264692; coumarin dyes; dicyanomethylenepyran dyes; dicyanomethylenethiopyran dyes; polymethine dyes; oxobenzanthracene dyes; xanthene dyes; carbostyryl dyes; perylene dyes; an oxazine compound described in German Patent No. 2534713; a stilbene derivative described in the Proceedings of the 40th Joint Lecture of Applied Physics, 1146 (1993); a spiro compound described in Japanese Patent Application (*kokai*) No. 7-278537; and an oxadiazole compound described in Japanese Patent Application (*kokai*) No. 4-393891.

Respective layers which constitute the organic EL device of the present invention may be produced by forming a thin film through a known method such as vapor deposition, spin-coating or casting and by use of materials for respective layers.

No particular limitation is imposed on the film thickness of the thus-obtained respective layers, and it may be selected in accordance with characteristics of the material. It is typically determined within the range of 2 nm-5000 nm.

In order to form thin film of a triphenylamine derivative, a vapor deposition method is preferably employed, in consideration that homogeneous film is easily produced and pinholes are difficult to generate. When thin film formation is preformed through a vapor deposition method, the desirable vapor deposition conditions are typically determined within the following ranges: boat heating temperature of 50-400°C, degree of vacuum of 10⁻⁶-10⁻³ Pa, vapor deposition rate of 0.01-50 nm/sec, substrate temperature of from -150 to +300°C, and film thickness of 5 nm-5 µm, in accordance with species of triphenylamine derivatives and crystal structure and association structure of target molecular accumulated films.

A method for producing an organic EL device using the triarylamine derivative of the present invention will next be described with reference to the case of an organic EL device formed of the above-described anode/triarylamine layer/cathode as an example. The target organic EL device is obtained through a method comprising the steps of fabricating an anode by forming a thin film comprising an anode material having a thickness of 1 µm or less, preferably 10-200 nm, on an appropriate substrate through vapor deposition; coating the anode with a thin film of triarylamine derivative, to thereby produce a luminescence layer; and coating the luminescence layer with a thin film comprising a cathode material so as to attain a film thickness of 1 µm or less, to thereby produce a cathode.

Alternatively, the above-described method may be performed in a reverse manner, i.e., successively forming a cathode, a luminescent layer, and an anode, in this sequence.

DC voltage is applied to the thus-obtained organic EL device, with polarity of the anode being positive (+) and the cathode negative (-). When the application voltage is 2-40 V, luminescence is observed through a transparent or opaque electrode (either anode or cathode, or both electrodes).

The organic EL device also generates luminescence when an AC voltage is applied thereto. In this case, the applied AC may have an arbitrary waveform.

### EXAMPLES

The present invention will next be described by way of example. Method for Measuring Glass Transition Point

Glass transition point (Tg) was measured by the following method. Briefly, a melted sample was quenched to have a glass state. Subsequently, the sample's temperature was elevated at a rate of 40°C/minute, and Tg was measured by a differential scanning calorimeter (DSC).

### Synthesis Example 1

### Synthesis of 1,4-bis{4-(N-naphthyl-N-phenylamino)phenyl}naphthalene (BPNAPN) (a compound represented by the above-mentioned formula (2)):

N-Phenyl-N-(1-naphthyl)-N-(4-bromophenyl)amine (2.0 g) and tetrahydrofuran (THF, 10 ml) were put into a flask and cooled to -78°C under nitrogen. Butyllithium (1.6 mol/l) in hexane (volume of the solution: 3.8 ml) was added dropwise to the flask while the flask was maintained at -70°C or less. The contents were stirred for 15 minutes. Subsequently, zinc chloride (1 mol/l) in ether (volume of the solution: 6 ml) was added to the mixture and the mixture was stirred for 1 hour at room temperature. Then, a THF solution (5 ml) containing 1,4-dibromonaphthalene (0.728 g) and dichlorobistriphenylphosphinepalladium (54 mg) were added thereto, and the resultant mixture was heated and refluxed for 24 hours. After the mixture was allowed to cool, water (50 ml) was added, and the solid matter was removed by filtration. Low-boiling-point substances were distilled off under reduced pressure. The residue was purified by silica gel column chromatography (eluant: heptane/ethyl acetate=10/1), followed by recrystallization from heptane-ethyl acetate, to thereby obtain 0.24 g of the title compound. The fluorescent color of the compound in toluene was bluish violet and Tg of the compound was 120°C.

### Synthesis Example 2

### Synthesis of 2,6-bis{4-N-naphthyl-N-phenylamino)phenyl}pyridine (a compound represented by the above-mentioned formula (4)):

The procedure of Synthesis Example 1 was repeated except that 2,6-dibromopyridine was used instead of 1,4-dibromonaphthalene, to thereby synthesize the title compound.
¹H-NMR(CDCl₃) δ=6.9-7.0(m,2H), 7.0-7.15(m,8H), 7.15-7.25(m,4H), 7.3-7.4(m,4H), 7.4-7.55(m,6H), 7.68(t,1H), 7.78(d,2H), 7.89(d,2H), 7.9-8.0(m,6H).

### Synthesis Example 3

### Synthesis of 2,6-bis{4-(N,N-diphenylamino)naphthyl}pyridine (a compound represented by the above-mentioned formula (13)):

The procedure of Synthesis Example 1 was repeated except that 2,6-dibromopyridine was used instead of 1,4-dibromonaphthalene, and that N-phenyl-N-(1-naphthyl)-4-bromoaniline was used instead of N-phenyl-N-(1-naphtyl)-N-(4-bromophenyl)amine. Tg of the compound was 132°C.
¹H-NMR(CDCl₃) δ=6.9-7.0(m,4H), 7.0-7.1(m,8H), 7.1-7.3(m,8H), 7.3-7.5(m,6H), 7.69(t,4H), 7.9-8.1(m,3H), 8.24(d,2H).

### Synthesis Example 4

### Synthesis of 2,6-bis{4-(N-naphthyl-N-phenylamino)phenyl}pyrimidine

The procedure of Synthesis Example 1 was repeated except that 2,6-dichloropyrimidine was used instead of 1,4-dibromonaphthalene, to thereby synthesize the title compound.
¹H-NMR(CDCl₃) δ=6.96-7.53(m,23H), 7.8(t,2H), 7.87-7.95(m,4H), 8.0(m,2H), 8.33(m,2H), 8.65(d,1H).

### Synthesis Example 5

### Synthesis of 3,6-bis{4-(N-naphthyl-N-phenylamino)phenyl}pyridazine

The procedure of Synthesis Example 1 was repeated except that 3,6-dichloropyridazine was used instead of 1,4-dibromonaphthalene, to thereby synthesize the title compound.
¹H-NMR(CDCl₃) δ=6.98-7.10(m,6H), 7.17(dd,4H), 7.22-7.39(m,4H), 7.34-7.55(m,8H), 7.75(s,2H), 7.82(d,2H), 7.88-8.0(m,8H).

### Synthesis Example 6

### Synthesis of 2,6-bis{4-(N-naphthyl-N-phenylamino)phenyl}pyradine

The procedure of Synthesis Example 1 was repeated except that 2,6-dichloropyradine was used instead of 1,4-dibromonaphthalene, to thereby synthesize the title compound.
¹H-NMR(CDCl₃) δ=6.99-7.0(m,6H), 7.15(dd,4H), 7.21-7.29(m,4H), 7.34-7.54(m,8H), 7.81(d,2H), 7.87-7.97(m,8H), 8.76(s,2H).

### Example 1

ITO was vapor-deposited to a thickness of 50 nm on a glass substrate (25 mm x 75 mm x 1.1 mm) (produced by Tokyo San'yoshinku K.K.), and the resultant glass was used as a transparent support substrate. The support substrate was fixed upon a substrate holder of a commercially available deposition apparatus (produced by Shinkukiko K.K.). A quartz crucible containing BPNAPN, a quartz crucible containing 1-allyl-1,2,3,4,5-pentaphenylsilacyclopentadiene (APS), a graphite crucible containing magnesium, and a graphite crucible containing silver were mounted on the apparatus.

The internal pressure of the vacuum vessel was reduced to 1 x 10⁻⁴ Pa. The crucible containing BPNAPN was heated for vapor deposition to form a hole-transportation layer on the support substrate, so as to attain a BPNAPN film thickness of 50 nm. Then, the crucible containing APS was heated for vapor deposition of APS thereon to form a luminescence layer having a APS film thickness of 50 nm. The vapor deposition rates were 0.1-0.2 nm/sec.

Subsequently, the internal pressure of the vacuum vessel was reduced to 2 x 10⁻⁴ Pa. The graphite crucible containing magnesium was heated for vapor deposition of magnesium at a deposition rate of 1.2-2.4 nm/sec, and simultaneously, silver was vapor-deposited at a rate of 0.1-0.2 nm/sec. Thereafter, an Mg-Ag alloy electrode (200 nm) was formed on the thus-formed organic layer, to thereby obtain an organic EL device.

When a DC voltage of 6.5 V was applied between the ITO electrode serving as a positive electrode and the Mg-Ag alloy electrode serving as a negative electrode, a current of about 20 mA/cm² flowed and green light having a luminance of about 200 cd/m² and a wavelength of 503 nm was emitted.

When a DC voltage of 6.5 V was continually applied at 70°C, emission of light continued even after one hour.

### Example 2

The procedure of Example 1 was repeated except that tris(8-hydroxyquinoline)aluminum (ALQ) was used instead of APS, to thereby obtain an organic EL device.

When a DC voltage of 5 V was applied between the ITO electrode serving as a positive electrode and the Mg-Ag alloy electrode serving as a negative electrode, a current of about 6 mA/cm² flowed and green light having a peak wavelength of 503 nm was obtained.

### Example 3

The vapor deposition device used in Example 1 was used to successively form, under vacuum (1 x 10⁻⁴ Pa), organic layers on a transparent support substrate having an ITO electrode (50 nm): as a hole injection layer, a BPNAPN layer having a film thickness of 10 nm; as a hole-transportation layer on the BPNAPN layer, a TPD layer having a film thickness of 40 nm; and as a luminescence layer on the TPD layer, an ALQ layer having a film thickness of 50 nm. The vapor deposition rates were 0.1-0.2 nm/sec.

Under the same conditions as used in Example 1, an Mg-Ag alloy electrode (film thickness: 200 nm) was formed on the organic layer to obtain an organic EL device.

When a DC voltage of 5 V was applied between the ITO electrode serving as a positive electrode and the Mg-Ag alloy electrode serving as a negative electrode, a current of about 5 mA/cm² flowed and green light of about 150 cd/m² was emitted.

### Example 4

The vapor deposition device used in Example 1 was used to perform a series of vapor depositions successively on a transparent support substrate having an ITO electrode (50 nm) under vacuum (1 x 10⁻⁴ Pa): a TPD layer having a film thickness of 50 nm so as to serve as a hole transporting layer; BPNAPN was deposited thereon to have a film thickness of 20 nm so as to serve as a luminescence layer; and 9,9'-spirobisilafluorene was deposited thereon to have a film thickness of 50 nm so as to serve as an electron-transportation layer. The vapor deposition rates were 0.1-0.2 nm/sec.

Under the same conditions as in Example 1, an Mg-Ag alloy electrode (film thickness: 200 nm) was formed on the organic layer in order to obtain an organic EL device.

When a DC voltage of 10 V was applied between the ITO electrode serving as a positive electrode and the Mg-Ag alloy electrode serving as a negative electrode, a current of about 50 mA/cm² flowed and the BPNAPN layer glowed blue.

### Example 5

The vapor deposition device used in Example 1 was used to form, under vacuum (1 x 10⁻⁴ Pa), an organic layer on a transparent support substrate having an ITO electrode (50 nm) by a series of vapor depositions in a successive manner: 4,4',4''-tris{N-(3-methylphenyl)-N-phenylamino}triphenylamine was deposited to have a film thickness of 30 nm so as to serve as a hole injection layer; BPNAPN was deposited thereon to have a film thickness of 20 nm so as to serve as a hole-transportation layer; and 2,5-bis{5-(2-benzo[b]thienyl)thienyl}-1,1,3,4-tetraphenylsilacyclo-pentadiene was deposited on the BPNAPN layer to have a film thickness of 50 nm so as to serve as a luminescence layer on the BPNAPN layer. The vapor deposition rates were 0.1-02 nm/sec.

Under the same conditions as Example 1, an Mg-Ag alloy electrode (film thickness: 200 nm) was formed on the organic layer to obtain an organic EL device.

When a DC voltage of 10 V was applied between the ITO electrode serving as a positive electrode and the Mg-Ag alloy electrode serving as a negative electrode, a current of about 100 mA/cm² flowed and red light was emitted.

### Example 6

The procedure of Example 1 was repeated except that 2,6-bis{4-(naphtyl-N-phenylamino)phenyl}pyridine was used instead of BPNAPN, to thereby obtain an organic EL device.

When a DC voltage of 5 V was applied between the ITO electrode serving as a positive electrode and the Mg-Ag alloy electrode serving as a negative electrode, a current of about 5 mA/cm² flowed and green light having a peak wavelength of 530 nm was obtained.

### Example 7

The procedure of Example 1 was repeated except that 2,5-bis{4-(N-naphtyl-N-phenylamino)phenyl}pyrimidine was used instead of BPNAPN, to thereby obtain an organic EL device.

When a DC voltage of 5.5 V was applied between the ITO electrode serving as a positive electrode and the Mg-Ag alloy electrode serving as a negative electrode, a current of about 7 mA/cm² flowed and green light having a peak wavelength of 530 nm was emitted.

### Example 8

The procedure of Example 1 was repeated except that 2,6-bis{4-(N,N-diphenylamino)naphthyl}pyridine was used instead of BPNAPN, to thereby obtain an organic EL device.

When a DC voltage of 5 V was applied between the ITO electrode serving as a positive electrode and the Mg-Ag alloy electrode serving as a negative electrode, a current of about 5 mA/cm² flowed and green light having a peak wavelength of 530 nm was obtained.

### Comparative Example 1

The procedure of Example 1 was repeated except that TPD was used instead of BPNAPN, to thereby obtain an organic EL device.

When a DC voltage of 7 V was applied between the ITO electrode serving as a positive electrode and the Mg-Ag alloy electrode serving as a negative electrode, a current of about 20 mA/cm² flowed and green light of 200 cd/m² was emitted. The wavelength of the light was 503 nm.

However, emission of light ended after several seconds of continuous application of a DC voltage (7 V). Also, Tg of TPD was 69°C.

### Comparative Example 2

The procedure of Example 1 was repeated except that N,N'-diphenyl-N,N'-(3-methylphenyl)-4,4'-diaminoterphenyl was used instead of BPNAPN, to thereby obtain an organic EL device.

When a DC voltage of 5 V was applied between the ITO electrode serving as a positive electrode and the Mg-Ag alloy electrode serving as a negative electrode, a current of about 1 mA/cm² flowed and green light of 200 cd/m² having a luminance of 20 cd/m² and a wavelength of 503 nm was obtained. This organic EL device had a wavelength of 503 nm as attained in Example 1; however, it had low luminance and poor efficiency.

## Claims

1. A luminescent material comprising a triarylamine derivative of formula (1): 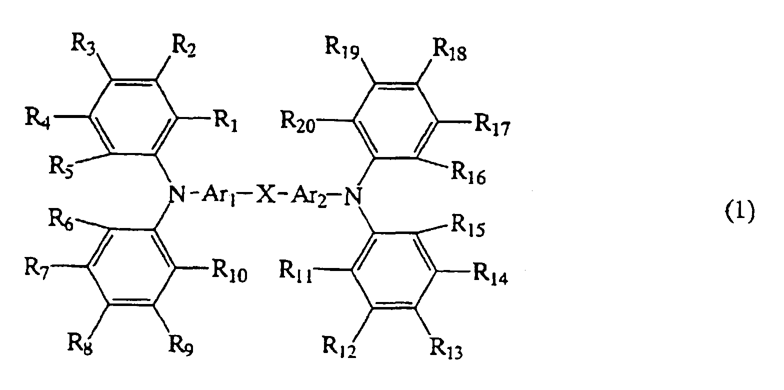 wherein X represents an arylene group other than a phenylene group, or a divalent heterocyclic group other than a thienylene group; Ar₁ and Ar₂ independently and individually represent a phenylene group or a naphthylene group; and R₁ through R₂₀ independently and individually represent a hydrogen atom, a halogen atom, a C1-C6 alkyl group, a C1-C6 alkoxy group, a substituted or unsubstituted amino group, a substituted or unsubstituted aryl group, or a substituted or unsubstituted heterocyclic group, the substituted or unsubstituted aryl group or the substituted or unsubstituted heterocyclic group may be condensed with the corresponding benzen ring at an arbitrary position of the benzen ring.

2. An organic electroluminescent material formed of the triarylamine derivative as described in Claim 1.

3. An organic electroluminescent device comprising the triarylamine derivative as described in Claim 1.

4. An organic electroluminescent device comprising a hole-transportation layer, wherein the hole-transportation layer contains the triarylamine derivative as described in Claim 1.

5. An organic electroluminescent device comprising a luminescence layer, wherein the luminescence layer contains the triarylamine derivative as described in Claim 1.

6. An organic electroluminescent device comprising a hole-injection layer, wherein the hole-injection layer contains the triarylamine derivative as described in Claim 1.
